# EUROPEAN PATENT APPLICATION

(11) **EP 4 818 148 A1**
(43) Date of publication of application: **07.10.2026**
(21) Application number: 24897624.3
(22) Date of filing: 28.11.2024
(51) Int. Cl.: C07C 63/333, B82Y 35/00, C07C 233/81, C07D 213/38, C07D 213/75, C07F 3/06, G01N 23/20025, G01N 23/207

(54) **THREE-DIMENSIONAL NETWORK STRUCTURED METAL COMPLEX CRYSTAL AND METHOD FOR DETERMINING MOLECULAR STRUCTURE OF ORGANIC COMPOUND USING SAME**

(30) Priority: 30.11.2023 JP 2023202762
(71) Applicant: The University of Tokyo, Bunkyo-ku, Tokyo 113-8654 (JP); Daicel Corporation, Osaka-shi, Osaka 530-0011 (JP)
(72) Inventor: SATO, Sota, Tokyo 113-8654 (JP); FUJITA, Makoto, Tokyo 113-8654 (JP); GONDO, Keisuke, Tokyo 108-8230 (JP)
(74) Representative: Grünecker Patent- und Rechtsanwälte PartG mbB
(86) International application number: PCT/JP2024/042082
(87) International publication number: WO 2025/115940

(57) **Abstract**

Provided is a novel metal complex crystal that has regularly arranged cavities and that can form an X-ray crystallographic sample by fixing an organic compound in the cavities. The metal complex crystal of the present disclosure comprises a metal ion and a ligand, and the ligand contains a carboxylic acid-based ligand (c) and a pyridine-based ligand (p) in a combination selected from [I] to [IV]: [I] a compound (c1) and a compound (p1), [II] a compound (c1) and a compound (p2), [III] a compound (c2) and a compound (p2), and [IV] a compound (c2) and a compound (p3).

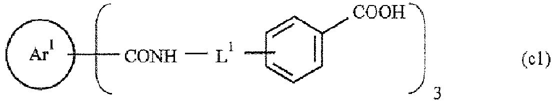

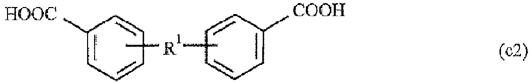

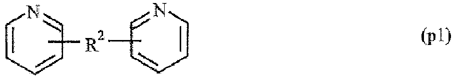

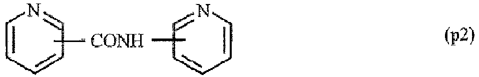

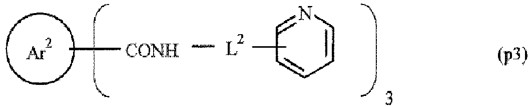

## Description

### Technical Field

The present disclosure relates to a novel three-dimensional network structured metal complex crystal, and a method for determining a molecular structure of an organic compound, the method using the three-dimensional network structured metal complex crystal. The present disclosure claims priority from JP 2023-202762 filed in Japan on November 30, 2023, the contents of which are incorporated herein by reference.

### Background Art

As a method for determining a molecular structure of an organic compound, single crystal X-ray crystallography, that is, a method for subjecting a single crystal of the organic compound to X-ray crystallography, has been known. This method is significantly useful because the molecular structure of the organic compound can be accurately determined if a high quality single crystal of the organic compound can be prepared.

However, when the organic compound is in a very small amount and a sufficient amount of single crystal cannot be obtained, determination of the molecular structure by this method has been difficult. Furthermore, an organic compound that hardly crystallizes exists, and this method could not determine a molecular structure of such an organic compound.

Thus, a crystalline sponge method that can determine the molecular structure of an organic compound by X-ray crystallography without crystallizing the organic compound has been developed (e.g., Patent Document 1). In the crystalline sponge method, a metal complex crystal having regularly arranged cavities is used as a material for forming an X-ray crystallographic sample (or a crystalline sponge or a host). In the crystalline sponge method, the metal complex crystal is impregnated with, as a guest molecule, an organic compound whose molecular structure is desired to be determined and a solvent, the organic compound is fixed in cavities of the metal complex crystal as a host, and thus a material having the regularly arranged organic compound is formed as an X-ray crystallographic sample (i.e., a sample subjected to the X-ray crystallography), and the obtained X-ray crystallographic sample is irradiated with X-rays to determine the molecular structure of the organic compound.

### Citation List

### Patent Document

Patent Document 1: WO 2014/038220

### Summary of Invention

### Technical Problem

However, in the metal complex crystal described in Patent Document 1, the probability of successful determination of the molecular structure of the organic compound is approximately 30%, and thus the low success rate is problematic.

Furthermore, although the success rate of the molecular structure determination can be increased by optimizing conditions, such as type of a solvent to be used during impregnation of the organic compound into the metal complex crystal or impregnation temperature, based on the type of the organic compound, the metal complex crystal has low solvent resistance and dissolves in moderately polar to highly polar solvents, and thus, there has been a problem of the limited number of types of solvents that can be used.

Furthermore, because the inside of each of the cavities of the metal complex crystal is in a hydrophobic environment, it is difficult for the cavities of the metal complex crystal to hold a hydrophilic organic compound, and determination of the molecular structure of the hydrophilic organic compound using the metal complex crystal has been difficult.

An object of the present disclosure is to provide a novel metal complex crystal having regularly arranged cavities and enabling formation of an X-ray crystallographic sample by fixing an organic compound in the cavities.

Another object of the present disclosure is to provide a novel metal complex crystal having regularly arranged cavities and, by using the metal complex crystal containing a fixed organic compound in the cavities as an X-ray crystallographic sample, enabling determination of molecular structures of various organic compounds with a high probability.

Another object of the present disclosure is to provide a method for determining a molecular structure of an organic compound, the method using the metal complex crystal.

### Solution to Problem

As a result of diligent research to solve the problems described above, the present inventors have discovered the following.

To determine a molecular structure of an organic compound, the organic compound needs to be regularly arranged; and however, in the metal complex crystal described in Patent Document 1, the organic compound is fixed in the cavities by relatively weak intermolecular interaction, such as Π-Π interaction or σ-Π interaction, and thus the probability of holding the organic compound in the cavities is low. It was found that the metal complex crystal described in Patent Document 1 has a weak function of regularly arranging an organic compound, and thus, when the metal complex crystal is used as a crystalline sponge, the probability of successful determination of the molecular structure is low.

Meanwhile, it was found that an amide group can undergo a strong interaction with various functional groups due to hydrogen bonding; when a particular ligand having an amide group is coordinated with a metal ion, a metal complex crystal having regularly arranged cavities can be obtained; the regularly arranged cavities of the metal complex crystal thus obtained can house an organic compound with a high probability and the housed organic compound is firmly held in an oriented state, and thus the molecular structure can be determined with a high probability by an X-ray diffraction method when the metal complex crystal containing the organic compound incorporated and fixed in the cavities is used as an X-ray crystallographic sample. The present disclosure was completed based on these findings.

Thus, the present disclosure provides a metal complex crystal,
the metal complex crystal containing a metal ion and a ligand coordinated to the metal ion,
the metal complex crystal having a three-dimensional network structure having regularly arranged cavities,
the ligand containing a carboxylic acid-based ligand (c) and a pyridine-based ligand (p), and
the carboxylic acid-based ligand (c) and the pyridine-based ligand (p) being in a combination selected from [I] to [IV]:
   [I] a compound represented by Formula (c1) and a compound represented by Formula (p1);
   [II] a compound represented by Formula (c1) and a compound represented by Formula (p2);
   [III] a compound represented by Formula (c2) and a compound represented by Formula (p2); and
   [IV] a compound represented by Formula (c2) and a compound represented by Formula (p3): 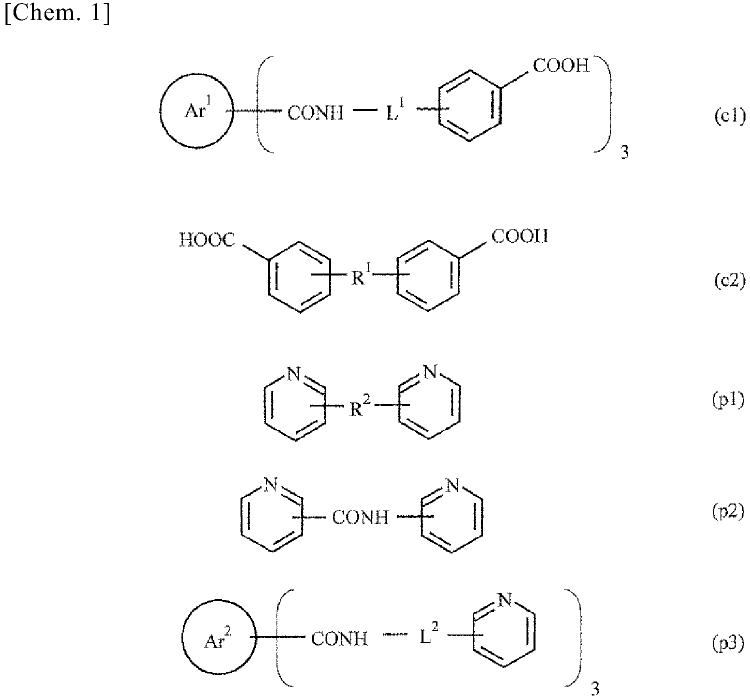
where, Ar¹ and Ar² are the same or different and each represent an aromatic ring; R¹ and R² are the same or different and each represent a group selected from a single bond, a divalent hydrocarbon group, a divalent heterocyclic group, or a divalent group containing two or more of the groups, the two or more of the groups being linked, and L¹ and L² are the same or different and each represent a single bond or a linking group.

The present disclosure also provides the metal complex crystal, wherein the metal ion is a metal ion selected from a zinc ion, an iron ion, a cobalt ion, a nickel ion, a copper ion, or a silver ion.

The present disclosure also provides the metal complex crystal, wherein the metal complex crystal is a metal complex crystal for molecular structure analysis of an organic compound.

The present disclosure also provides the metal complex crystal, wherein the organic compound is an organic compound including a functional group capable of interacting with a CO group, an NH group, or a CONH group.

The present disclosure also provides the metal complex crystal, wherein the organic compound is an organic compound having a log P value of -5 or greater and 7 or less.

The present disclosure also provides the metal complex crystal, wherein the organic compound is an organic compound including at least one functional group selected from a substituted or unsubstituted amino group, a carboxyl group, an active methylene group, a nitrile group, a ketone group, an aldehyde group, an ester group, an ether group, an amide group, a hydroxy group, a halogen group, or a sulfonamide group.

The present disclosure also provides a method for determining a molecular structure of an organic compound (X), the method including steps 1 and 2:
step 1: allowing the organic compound (X) to infiltrate into the metal complex crystal, and fixing the organic compound (X) in cavities of the metal complex crystal; and
step 2: irradiating the metal complex crystal containing the fixed organic compound (X) with X-rays, analyzing obtained diffraction data, and determining a molecular structure of the organic compound (X).

### Advantageous Effects of Invention

The metal complex crystal of the present disclosure has large, regularly arranged cavities. Furthermore, the metal complex crystal includes an amide group, and the amide group is a group having a property of forming hydrogen bonds with various functional groups. Thus, when an organic compound is allowed to infiltrate into the cavities of the metal complex crystal, the organic compound can be firmly fixed with a high probability in a regularly arranged state.

Furthermore, because the metal complex crystal has an amide group, the cavities exhibit hydrophilic environments, and a hydrophilic organic compound can be fixed in the cavities.

Furthermore, the metal complex crystal has excellent solvent resistance, and can maintain the crystal structure in various solvents.

Accordingly, when the metal complex crystal is used as a host, even when the amount of the organic compound (X) as a guest molecule is too small to form a single crystal or even when the organic compound (X) is a compound that cannot form a single crystal, the organic compound (X) is fixed in the metal complex crystal in a regularly arranged manner and the organic compound (X) is subjected to an X-ray crystallographic treatment, and thus, the molecular structure can be easily determined with a high probability.

Because the metal complex crystal has the characteristics described above, the metal complex crystal is highly versatile as a host in the crystalline sponge method. More specifically, the metal complex crystal is highly versatile as a material for forming an X-ray crystallographic sample for an organic compound (X) (especially, a hydrophilic organic compound).

### Brief Description of Drawings

FIG. 1 is a schematic view illustrating a crystal structure of a metal complex crystal 1 obtained in Example.
FIG. 2 is a schematic view illustrating a crystal structure of a metal complex crystal 3 obtained in Comparative Example.
FIG. 3 is a schematic view illustrating a state where 4-methylacetophenone is fixed to the metal complex crystal 1 by hydrogen bonding.
FIG. 4 is a schematic view illustrating a state where 1,3-dimethyl-2-imidazolidinone is fixed to the metal complex crystal 1 by hydrogen bonding.
FIG. 5 is a schematic view illustrating a state where methyl 2-furancarboxylate is fixed to the metal complex crystal 1 by hydrogen bonding.
FIG. 6 is a schematic view illustrating a state where 2,4-dimethoxybenzyl alcohol is fixed to the metal complex crystal 1 by hydrogen bonding.
FIG. 7 is a schematic view illustrating a state where 2-naphthalenecarboxylic acid is fixed to the metal complex crystal 1 by hydrogen bonding.
FIG. 8 is a schematic view illustrating a state where zonisamide is fixed to the metal complex crystal 1 by hydrogen bonding.
FIG. 9 is a schematic view illustrating a state where 2,4,6-trimethylaniline is fixed to the metal complex crystal 1 by hydrogen bonding.
FIG. 10 is a schematic view illustrating a state where bergapten is fixed to the metal complex crystal 1 by hydrogen bonding.
FIG. 11 is a schematic view illustrating a state where two molecules of bergapten are fixed to the metal complex crystal 1 by Π-Π interaction.
FIG. 12 is a schematic view illustrating a state where 1-aminopyrene is fixed to the metal complex crystal 1 by Π-Π interaction.

### Description of Embodiments

### Metal Complex Crystal

The metal complex crystal of the present disclosure is a crystal of a metal complex containing a metal ion and a ligand coordinated to the metal ion.

The metal complex crystal is a microporous metal complex crystal having a three-dimensional network structure having regularly arranged cavities.

The metal ion is not particularly limited as long as such a three-dimensional network structure can be formed by a coordinate bond of a ligand described below. Examples of the metal ion include ions of metals of Groups 8 to 12 in the periodic table, such as a zinc ion, an iron ion, a cobalt ion, a nickel ion, a copper ion, and a silver ion. In particular, from the viewpoint of yielding a three-dimensional network structured metal complex crystal having large cavities, a divalent metal ion is preferred, and a zinc ion and/or a cobalt ion is particularly preferred.

The ligand contains at least one carboxylic acid-based ligand (c) and at least one pyridine-based ligand (p).

The combination of the carboxylic acid-based ligand (c) and the pyridine-based ligand (p) is a combination selected from [I] to [IV]:
[I] a compound represented by Formula (c1) and a compound represented by Formula (p1);
[II] a compound represented by Formula (c1) and a compound represented by Formula (p2);
[III] a compound represented by Formula (c2) and a compound represented by Formula (p2); and
[IV] a compound represented by Formula (c2) and a compound represented by Formula (p3): 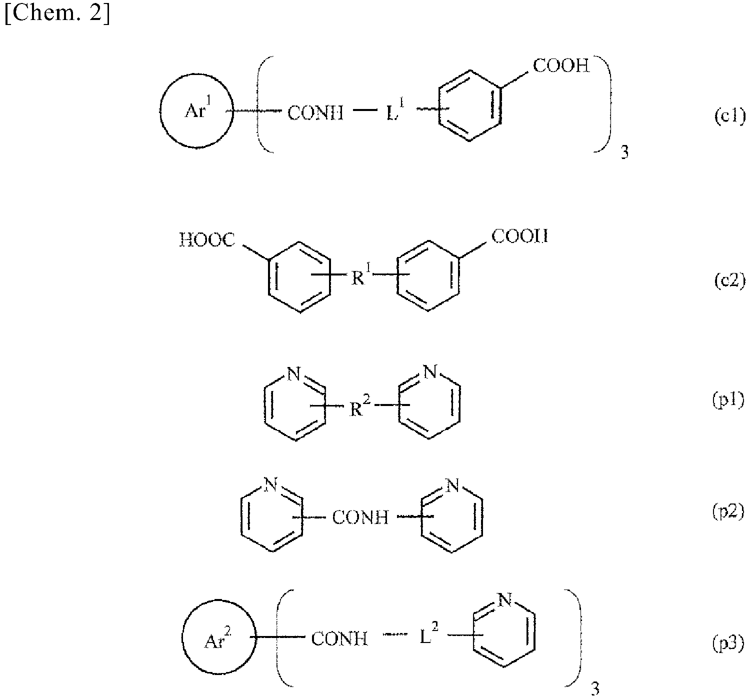
where, Ar¹ and Ar² are the same or different and each represent an aromatic ring; R¹ and R² are the same or different and each represent a group selected from a single bond, a divalent hydrocarbon group, a divalent heterocyclic group, or a divalent group containing two or more of the groups, the two or more of the groups being linked, and L¹ and L² are the same or different and each represent a single bond or a linking group.

The aromatic ring of Ar¹ and Ar² includes an aromatic hydrocarbon ring and an aromatic heterocyclic ring.

Examples of the aromatic hydrocarbon ring include 6-membered aromatic hydrocarbon rings, such as a benzene ring, a phenalene ring, and a phenanthrene ring, and condensed rings in which two or more of the 6-membered aromatic hydrocarbon rings are fused.

Examples of the aromatic heterocyclic ring include 6-membered aromatic heterocyclic rings containing a nitrogen atom as a heteroatom, such as a pyridine ring, a pyrimidine ring, and a 1,3,5-triazine ring.

From the viewpoint of yielding a three-dimensional network structured metal complex crystal having large cavities, the aromatic ring of Ar¹ and Ar² is preferably a 6-membered aromatic ring, and particularly preferably a benzene ring or a 1,3,5-triazine ring.

Examples of the divalent hydrocarbon group of R¹ and R² include linear or branched alkylene groups having from 1 to 18 carbons, such as a methylene group, a methylmethylene group, a dimethylmethylene group, an ethylene group, a propylene group, and a trimethylene group; cycloalkylene groups having from 3 to 18 carbons, such as a 1,2-cyclopentylene group, a 1,3-cyclopentylene group, a cyclopentylidene group, a 1,2-cyclohexylene group, a 1,3-cyclohexylene group, a 1,4-cyclohexylene group, and a cyclohexylidene group; and C₆₋₁₄ arylene groups, such as a phenylene group (e.g., an o-phenylene group, an m-phenylene group, and a p-phenylene group), a phenylenebis(methylene) group (e.g., a 1,2-phenylenebis(methylene) group, a 1,3-phenylenebis(methylene) group, and a 1,4-phenylenebis(methylene) group), a biphenylene group, a naphthylene group, a binaphthylene group, an anthracenylene group, and a phenanthrenylene group.

The divalent heterocyclic group of R¹ and R² is a group having a structural formula of a heterocyclic ring with two hydrogen atoms removed. Examples of the heterocyclic ring include an aromatic heterocyclic ring and a non-aromatic heterocyclic ring. Examples of such a heterocyclic ring include 3 to 20-membered rings (preferably 3 to 10-membered rings and particularly preferably 4 to 6-membered rings) having carbon atoms and at least one heteroatom (e.g., an oxygen atom, a sulfur atom, or a nitrogen atom) as ring atoms, and condensed rings thereof. Specific examples thereof include heterocyclic rings containing an oxygen atom as a heteroatom (e.g., 3-membered rings, such as an oxirane ring; 4-membered rings, such as an oxetane ring; 5-membered rings, such as a furan ring, a tetrahydrofuran ring, an oxazole ring, an isoxazole ring, and a γ-butyrolactone ring; 6-membered rings, such as a 4-oxo-4H-pyran ring, a tetrahydropyran ring, and a morpholine ring; condensed rings, such as a benzofuran ring, an isobenzofuran ring, a 4-oxo-4H-chromene ring, a chroman ring, and an isochroman ring; and bridged rings, such as a 3-oxatricyclo[4.3.1.1^{4,8}]undecan-2-one ring and a 3-oxatricyclo[4.2.1.0^{4,8}]nonan-2-one ring), heterocyclic rings containing a sulfur atom as a heteroatom (e.g., 5-membered rings, such as a thiophene ring, a thiazole ring, an isothiazole ring, and a thiadiazole ring; 6-membered rings, such as a 4-oxo-4H-thiopyran ring; and condensed rings, such as a benzothiophene ring), and heterocyclic rings containing a nitrogen atom as a heteroatom (e.g., 5-membered rings, such as a pyrrole ring, a pyrrolidine ring, a pyrazole ring, an imidazole ring, and a triazole ring; 6-membered rings, such as an isocyanuric ring, a pyridine ring, a pyridazine ring, a pyrimidine ring, a pyrazine ring, a piperidine ring, and a piperazine ring; and condensed rings, such as an indole ring, an indoline ring, a quinoline ring, an acridine ring, a naphthyridine ring, a quinazoline ring, and a purine ring).

L¹ and L² are the same or different and each represent a single bond or a linking group. The linking group is a divalent group having one or more atoms, and examples thereof include a divalent hydrocarbon group.

Examples of the divalent hydrocarbon group include linear or branched alkylene groups having from 1 to 18 carbons, such as a methylene group, a methylmethylene group, a dimethylmethylene group, an ethylene group, a propylene group, and a trimethylene group; cycloalkylene groups having from 3 to 18 carbons, such as a 1,2-cyclopentylene group, a 1,3-cyclopentylene group, a cyclopentylidene group, a 1,2-cyclohexylene group, a 1,3-cyclohexylene group, a 1,4-cyclohexylene group, and a cyclohexylidene group; C₆₋₁₄ arylene groups, such as a phenylene group (e.g., an o-phenylene group, an m-phenylene group, and a p-phenylene group), a naphthylene group (e.g., a 1,3-naphthylene group, a 1,4-naphthylene group, a 1,5-naphthylene group, and a 2,7-naphthylene group), an anthracenylene group, and a phenanthrenylene group; and a group in which two or more thereof are linked [e.g., a phenylenebis(methylene) group (e.g., a 1,2-phenylenebis(methylene) group, a 1,3-phenylenebis(methylene) group, and a 1,4-phenylenebis(methylene) group), a biphenylene group, and a binaphthylene group].

The divalent hydrocarbon group is preferably a C₆₋₁₄ arylene group or a group in which two or more of the arylene groups are bonded through a single bond or a C₁₋₅ alkylene group, particularly preferably a phenylene group or a naphthylene group, and most preferably a phenylene group.

The benzene ring and the pyridine ring in Formulas (c1), (c2), (p1), (p2), and (p3) above may have a substituent in addition to the groups represented in Formulas.

Examples of the substituent include halogen atoms, oxo groups, hydroxyl groups, substituted oxy groups (e.g., C₁₋₄ alkoxy groups), cyano groups, nitro groups, substituted or unsubstituted amino groups (e.g., mono- or di-C₁₋₄ alkylamino groups, such as a methylamino group, a dimethylamino group, an ethylamino group, and a diethylamino group; 5 to 8-membered cyclic amino groups, such as a 1-pyrrolidinyl group, a piperidino group, and a morpholino group; C₁₋₁₀ acylamino groups, such as an acetylamino group, a propionylamino group, and a benzoylamino group; and sulfonylamino groups, such as a benzenesulfonylamino group and a p-toluenesulfonylamino group), sulfonic acid groups, alkyl groups (e.g., C₁₋₄ alkyl groups, such as a methyl group and an ethyl group), alkenyl groups (e.g., C₂₋₄ alkenyl groups, such as a vinyl group, an allyl group, and a 1-butenyl group), alkynyl groups (e.g., C₂₋₄ alkynyl groups, such as an ethynyl group and a propynyl group), C₃₋₈ cycloalkyl groups, and aryl groups (e.g., C₆₋₁₀ aryl groups, such as a phenyl group and a naphthyl group).

When the benzene ring or the pyridine ring in Formulas (c1), (c2), (p1), (p2), and (p3) above has two or more substituents, the two groups selected from the substituents described above may be bonded together to form a ring.

Furthermore, an aromatic or non-aromatic hydrocarbon ring or heterocyclic ring may be fused to the benzene ring or the pyridine ring in Formulas (c1), (c2), (p1), (p2), and (p3) above.

Specific examples of the compound represented by Formula (c1) above include a compound represented by Formula (c1-1), a compound represented by Formula (c1-2), a compound represented by Formula (c1-3), and a compound represented by Formula (c1-4) as described below. The compounds represented by the formulas below include a compound, in which CONH groups (or CONH-L- groups) are bonded at the ortho-position, the meta-position, or the para-position with respect to the linking parts of the carboxyl groups of the benzene rings.

In the formulas below, L¹¹ to L¹³ each independently represent a linking group. The linking group is preferably a divalent hydrocarbon group, more preferably a C₆₋₁₄ arylene group or a group in which two or more of the arylene groups are bonded through a single bond or a C₁₋₅ alkylene group, particularly preferably a phenylene group or a naphthylene group, and most preferably a phenylene group.

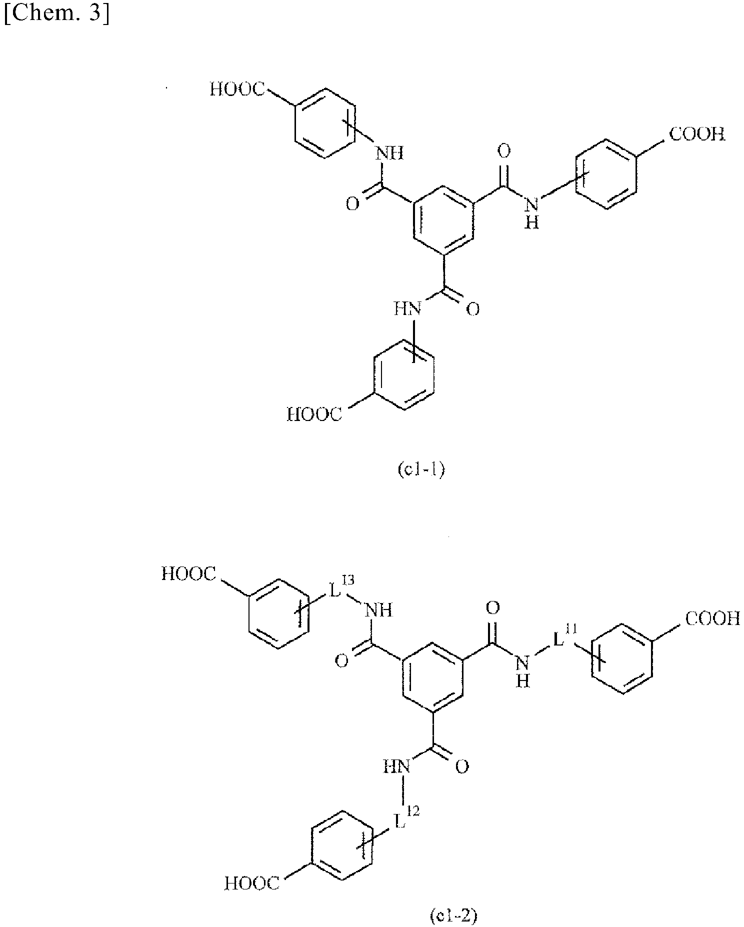

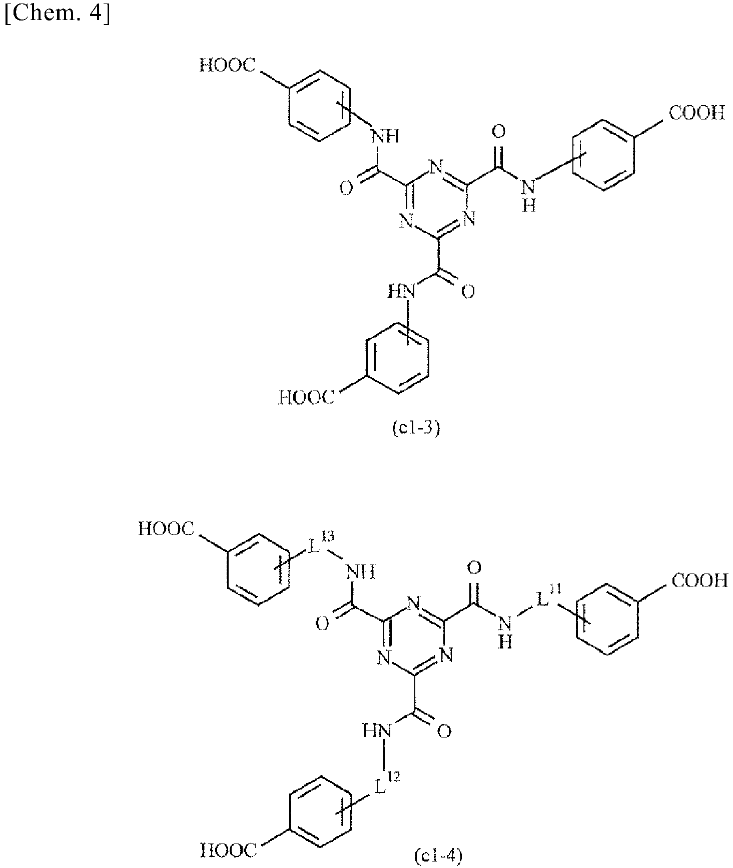

As the compound represented by Formula (c1) above, a compound represented by Formula (c1-1) above and a compound represented by Formula (c1-2) above are preferred, from the viewpoint of yielding a three-dimensional network structured metal complex crystal with large cavities.

As the compound represented by Formula (c1) above, a compound represented by Formula (c1-1) above and a compound represented by Formula (c1-3) above are preferred, from the viewpoint of yielding a three-dimensional network structured metal complex crystal with large cavities.

Specific examples of the compound represented by Formula (c2) include a compound represented by Formula (c2-1), a compound represented by Formula (c2-2), a compound represented by Formula (c2-3), a compound represented by Formula (c2-4), and a compound represented by Formula (c2-5) as described below. Examples of the compound represented by Formula (c2-1) below include a compound having a carboxyl group bonded at the ortho-position, the meta-position, or the para-position with respect to the linking part of the benzene rings. The same applies to the compounds represented by Formulas (c2-2) to (c2-5).

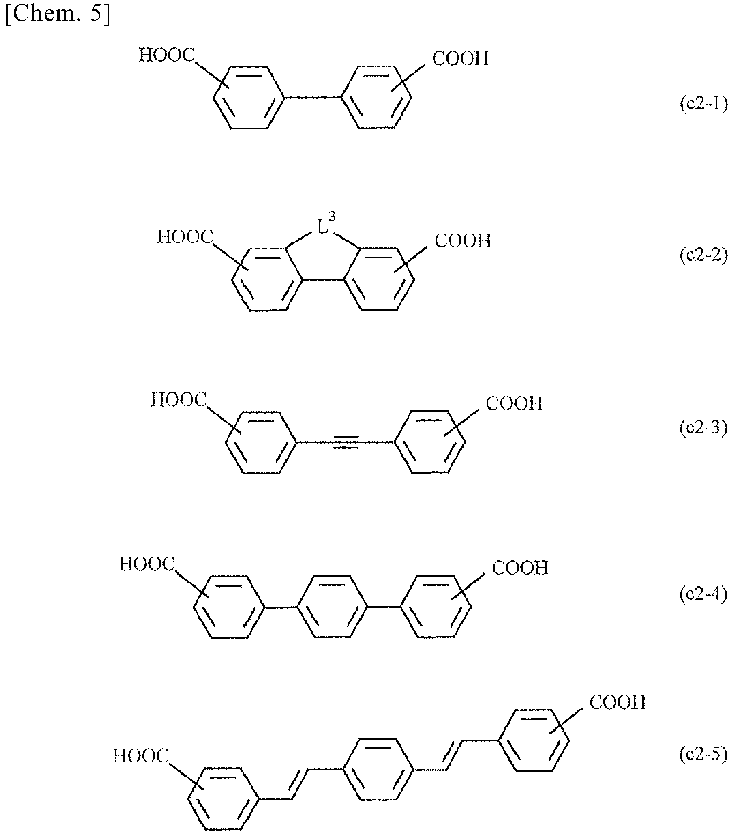

In Formula (c2-2) above, L³ represents a linking group. Examples of the linking group include a carbonyl group (-CO-), an ether bond (-O-), a thioether bond (-S-), an ester bond (-COO-), an amide group (-CONH-), an imino group (-NH-), and a sulfonyl group (-S(=O)₂-).

As the compound represented by Formula (c2) above, a compound represented by Formula (c2-1) above and a compound represented by Formula (c2-2) above are preferred, from the viewpoint of yielding a three-dimensional network structured metal complex crystal with large cavities.

Specific examples of the compound represented by Formula (p1) above include compounds represented by Formulas (p1-1) to (p1-7); and compounds, each corresponding to any one of the compounds represented by Formulas (p1-1) to (p1-7), each of the compounds having a group corresponding to the R² group in Formula (p1), bonded at the ortho-position or the meta-position with respect to the nitrogen atoms of the pyridine rings present on the both ends.

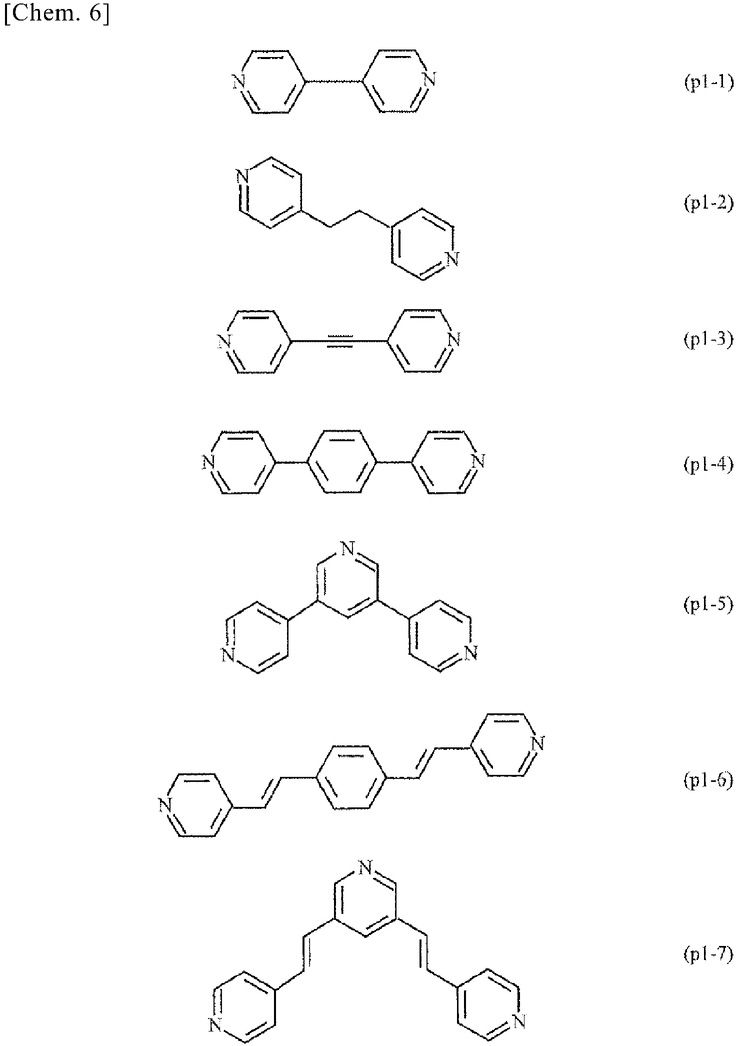

As the compound represented by Formula (p1) above, from the viewpoint of yielding a three-dimensional network structured metal complex crystal with large cavities, the compound represented by Formula (p1-1) above, the compound represented by Formula (p1-2) above, the compound represented by Formula (p1-5) above, and the compound represented by Formula (p1-6) above are preferred, the compound represented by Formula (p1-1) above and the compound represented by Formula (p1-2) above are particularly preferred, and the compound represented by Formula (p1-2) above is most preferred.

The compound represented by Formula (p2) above includes a compound, in which a CONH group bonds at the ortho-position, the meta-position, or the para-position with respect to the nitrogen atoms of the pyridine rings.

Specific examples of the compound represented by Formula (p3) above include a compound represented by Formula (p3-1), a compound represented by Formula (p3-2), a compound represented by Formula (p3-3), and a compound represented by Formula (p3-4). The compounds represented by the formulas below include a compound, in which the CONH groups (or the CONH-L groups) are bonded at the ortho-position, the meta-position, or the para-position with respect to the nitrogen atoms of the pyridine rings.

In the formulas below, L²¹ to L²³ each independently represent a linking group. The linking group is preferably a divalent hydrocarbon group, more preferably a C₆₋₁₄ arylene group or a group in which two or more of the arylene groups are bonded through a single bond or a C₁₋₅ alkylene group, particularly preferably a phenylene group or a naphthylene group, and most preferably a phenylene group.

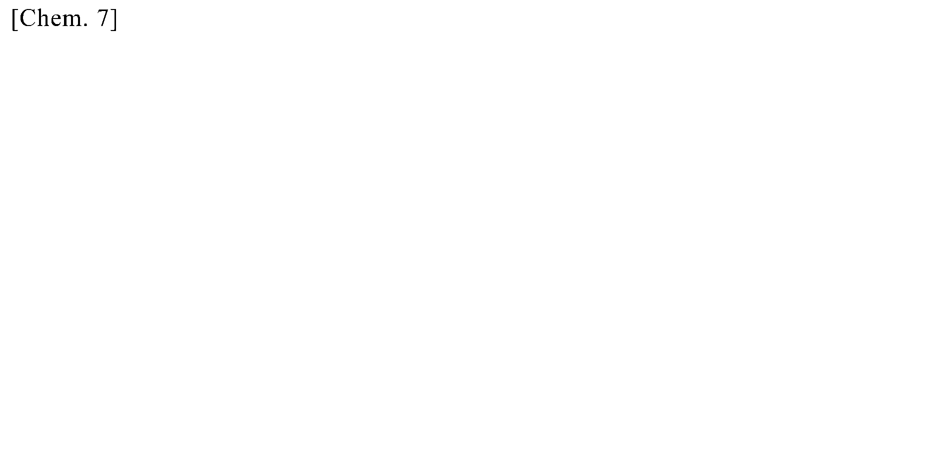

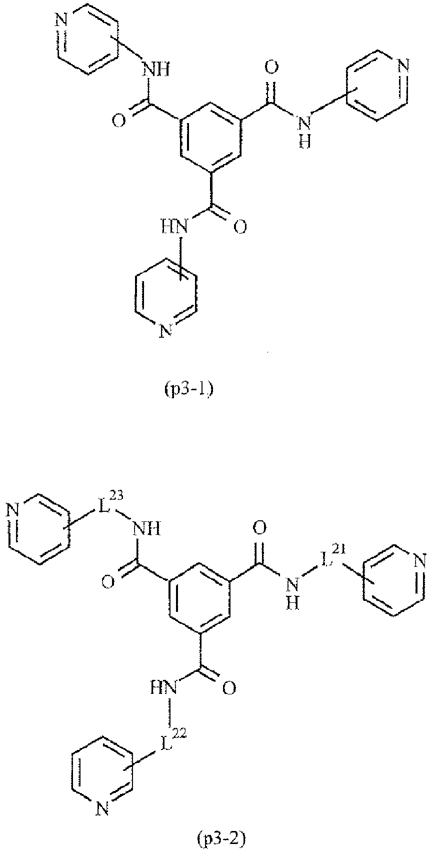

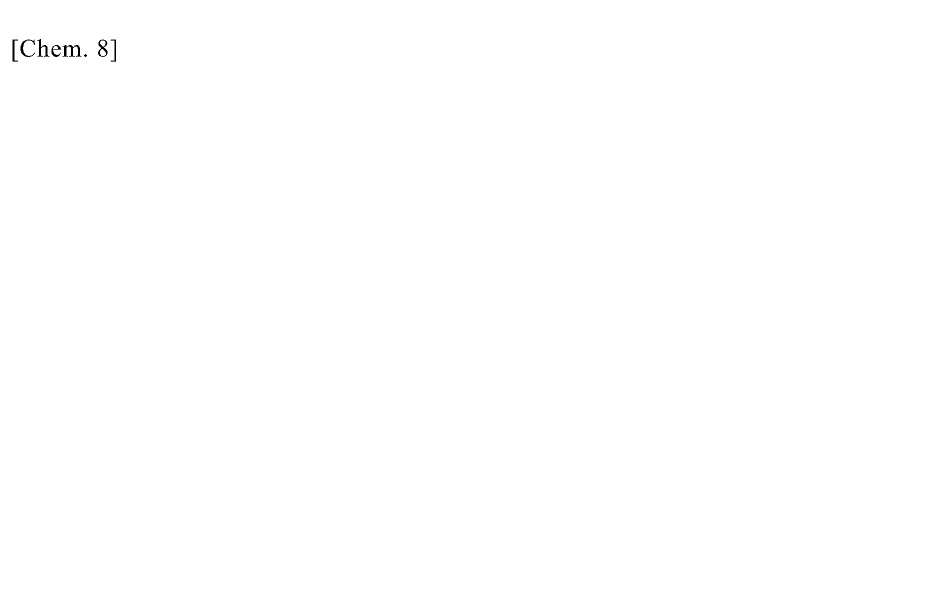

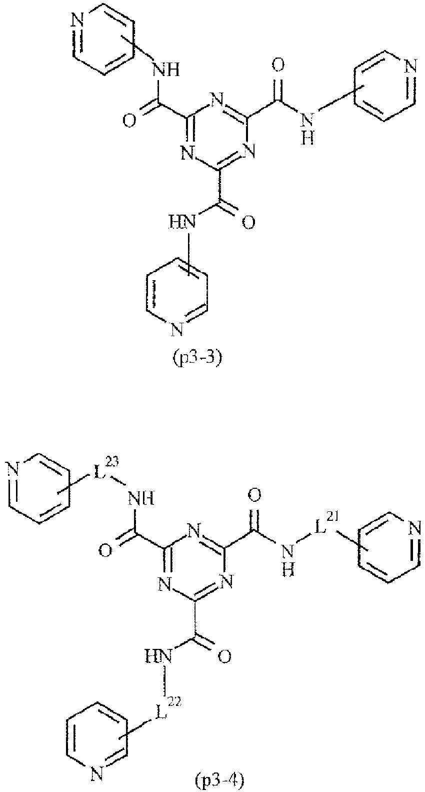

As the compound represented by Formula (p3) above, the compound represented by Formula (p3-1) above and the compound represented by Formula (p3-2) above are preferred from the viewpoint of yielding a three-dimensional network structured metal complex crystal with large cavities.

As the compound represented by Formula (p3) above, the compound represented by Formula (p3-1) above and a compound represented by Formula (p3-3) above are preferred from the viewpoint of yielding a three-dimensional network structured metal complex crystal with large cavities.

The ligand contained in the metal complex crystal contains the carboxylic acid-based ligand (c) and the pyridine-based ligand (p), and the content ratio of the carboxylic acid-based ligand (c) to the pyridine-based ligand (p) [carboxylic acid-based ligand (c)/pyridine-based ligand (p); molar ratio] is, for example, from 30/70 to 70/30, and, from the viewpoint of yielding a three-dimensional network structured metal complex crystal with large cavities, preferably from 40/60 to 60/40, and particularly preferably from 45/55 to 55/45.

When the ligand contains the compound represented by Formula (c1) above and the compound represented by Formula (p1) above, the content ratio of these compounds [compound represented by Formula (c1) above/compound represented by Formula (p1) above; molar ratio] is, for example, from 30/70 to 70/30, preferably from 40/60 to 60/40, and particularly preferably from 45/55 to 55/45.

When the ligand contains the compound represented by Formula (c1) above and the compound represented by Formula (p2) above, the content ratio of these compounds [compound represented by Formula (c1) above/compound represented by Formula (p2) above; molar ratio] is, for example, from 30/70 to 70/30, preferably from 40/60 to 60/40, and particularly preferably from 45/55 to 55/45.

When the ligand contains the compound represented by Formula (c2) above and the compound represented by Formula (p2) above, the content ratio of these compounds [compound represented by Formula (c2) above/compound represented by Formula (p2) above; molar ratio] is, for example, from 30/70 to 70/30, preferably from 40/60 to 60/40, and particularly preferably from 45/55 to 55/45.

When the ligand contains the compound represented by Formula (c2) above and the compound represented by Formula (p3) above, the content ratio of these compounds [compound represented by Formula (c2) above/compound represented by Formula (p3) above; molar ratio] is, for example, from 30/70 to 70/30, preferably from 40/60 to 60/40, and particularly preferably from 45/55 to 55/45.

The content ratio of the metal ion to the carboxylic acid-based ligand (c) in the metal complex crystal [metal ion/carboxylic acid-based ligand (c); molar ratio] is, for example, from 30/70 to 70/30, preferably from 40/60 to 60/40, and particularly preferably from 45/55 to 55/45.

The content ratio of the metal ion to the pyridine-based ligand (p) in the metal complex crystal [metal ion/pyridine-based ligand (p); molar ratio] is, for example, from 20/80 to 80/20, preferably from 25/75 to 60/40, particularly preferably from 30/70 to 45/55, and most preferably from 30/70 to 40/60.

The ligand may contain an additional ligand besides the carboxylic acid-based ligand (c) and the pyridine-based ligand (p). Examples of the additional ligand include anions such as F⁻, Cl⁻, Br⁻, I⁻, SCN⁻, NO₃⁻, ClO₄⁻, BF₄⁻, SbF₄⁻, PF₆⁻, AsF₆⁻, and CH₃COO⁻.

As the anions, from the viewpoint of yielding a three-dimensional network structured metal complex crystal with large cavities, an anion selected from NO₃⁻, F⁻, Cl⁻, Br⁻, or I⁻ is preferred, and NO₃⁻ is particularly preferred.

The proportion of the total content of the carboxylic acid-based ligand (c) and the pyridine-based ligand (p) with respect to the total amount of the ligand contained in the metal complex crystal is, for example, 50 wt.% or greater, preferably 60 wt.% or greater, more preferably 70 wt.% or greater, even more preferably 80 wt.% or greater, and particularly preferably 90 wt.% or greater.

The metal complex crystal has a three-dimensional network structure having regularly arranged cavities. Each of the cavities is a space present inside the metal complex crystal and is a space partitioned by a network structure made of metal ions and ligands.

The cavities are preferably regularly arranged three-dimensionally without irregularity to the degree that can be confirmed by X-ray crystallography.

The shapes of the pores are not particularly limited; and however, the respective cavities preferably have a fixed shape and a size to the degree that can be confirmed by X-ray crystallography.

The size of each of the cavities present in single unit cell preferably has a volume that can contain one to some moieties (e.g., from 1 to 6) of organic compounds (X).

The size of a cavity correlates with a diameter of an inscribed circle of the cavity on a plane parallel to the crystal plane that is as close as possible to being perpendicular to the extending direction of the cavity (hereinafter, such a plane may be referred to as "parallel plane"). Hereinafter, such an inscribed circle may be simply referred to as an "inscribed circle of the cavity". When the inscribed circle is larger, the cavity is larger. When the inscribed circle is smaller, the cavity is smaller.

The "extending direction of the cavity" can be determined by the following method.

First, a crystal plane 1 (one of six crystal planes connecting the four lattice points included in one unit cell) in a proper direction that intersects a target cavity is selected. A cross-sectional view of the cavity with the crystal plane 1 as a cutting plane is drawn by representing an atom that is contained in the metal complex crystal and is present on the crystal plane 1, using a van der Waals radius. Similarly, a cross-sectional view of the cavity is drawn using, as a cutting plane, a crystal plane 2 that is shifted by one unit cell from the crystal plane 1. Then, the centers of the cross-sectional shapes of the cavities of the respective crystal planes are connected by a straight line in a three-dimensional view. The direction of the straight line obtained at this time is the extending direction of the cavity.

Furthermore, the "diameter of the inscribed circle of the cavity" can be determined by the following method.

First, a cross-sectional view of the cavity is drawn by the method described above. Next, an inscribed circle is drawn in the cross-sectional view, and after the diameter of the inscribed circle is measured, the obtained measured value is converted into the actual scale, and thus, the actual diameter of the inscribed circle of the cavity can be determined.

Furthermore, while the parallel plane is gradually moved parallel within one unit cell, cross-sectional views of the cavity in the respective parallel planes are drawn, and by measuring the diameters of the inscribed circles, the diameter of the inscribed circle at the narrowest part and the diameter of the inscribed circle of the widest part can be determined.

The diameter of the inscribed circle of each of the cavities is, for example, from 8 to 12 Å, and preferably from 9 to 12 Å.

Furthermore, when the cavities have a shape significantly different from a perfect circle, the size of the respective cavities is preferably estimated based on the minor axis and the major axis of an inscribed ellipse of each of the cavities in the aforementioned parallel plane.

The major axis of the inscribed ellipse of each of the cavities is preferably from 10 to 15 Å, and more preferably from 10 to 12 Å. Furthermore, the minor axis of the inscribed ellipse of each of the cavities is preferably from 8 to 15 Å, and more preferably from 9 to 11 Å.

Although the metal complex crystal contains the ligand containing an amide group (CONH) as a constituent component, the amide group (CONH group) and the CO group and the NH group contained in the amide group can interact with various functional groups through hydrogen bonding. Thus, the metal complex crystal can determine molecular structures of a wide range of organic compounds or can determine molecular structures of many types of organic compounds. The metal complex crystal can firmly fix the organic compound in the cavities by a hydrogen bond with the amide group, the CO group, or the NH group. Thus, when the metal complex crystal is used, molecular structures of various organic compounds having functional groups that interact capable of interacting with the amide group, the CO group, or the NH group can be determined with a high probability.

Thus, the metal complex crystal can be suitably used as a material for forming an X-ray crystallographic sample for the organic compound in the crystalline sponge method (the so-called crystalline sponge) or a metal complex crystal for molecular structure analysis for the organic compound (i.e., the metal complex crystal used for molecular structure analysis of the organic compound by X-ray crystallography).

### Method for Producing Metal Complex Crystal

The metal complex crystal can be produced by, for example, reacting a carboxylic acid-based ligand (c), a pyridine-based ligand (p), and a salt of a metal ion and a counter ion (hereinafter, may be referred to as "metal salt") in the presence of a solvent.

Examples of the counter ion include anions such as F⁻, Cl⁻, Br⁻, I⁻, SCN⁻, NO₃⁻, ClO₄⁻, BF₄⁻, SbF₄⁻, PF₆⁻, AsF₆⁻, and CH₃COO⁻.

The used ratio of the carboxylic acid-based ligand (c) to the pyridine-based ligand (p) [carboxylic acid-based ligand (c)/pyridine-based ligand (p); molar ratio] is, for example, from 10/90 to 90/10. The lower limit value of the used ratio is preferably 15/85, and particularly preferably 20/80, from the viewpoint of yielding a three-dimensional network structured metal complex crystal with large cavities. The upper limit value of the used ratio is preferably 80/20, particularly preferably 60/40, most preferably 55/45, and especially preferably 45/55, from the viewpoint of yielding a three-dimensional network structured metal complex crystal with large cavities.

The used amount of the metal salt with respect to 1 mol of the carboxylic acid-based ligand (c) is, for example, from 0.5 to 3.5 mol, preferably from 0.5 to 2.0 mol, and particularly preferably from 0.8 to 1.5 mol.

Furthermore, the used amount of the metal salt with respect to 1 mol of the pyridine-based ligand (p) is, for example, from 0.2 to 3.5 mol, preferably from 0.3 to 2.0 mol, and particularly preferably from 0.4 to 1.0 mol.

Examples of the solvent include water; benzene; benzene derivatives obtained by substituting at least one hydrogen atom bonded to a benzene ring with a halogen atom, an alkyl group, a haloalkyl group, an alkoxy group, a cyano group, a nitro group, or a substituted oxycarbonyl group, such as toluene, xylene, ethylbenzene, trifluoromethylbenzene (or trifluorotoluene), chlorobenzene, anisole, benzonitrile, nitrobenzene, and ethyl benzoate; aliphatic hydrocarbons, such as hexane, heptane, and octane; alicyclic hydrocarbons, such as cyclohexane; haloalkane, such as carbon tetrachloride, chloroform, dichloromethane, and 1,2-dichloroethane; ketones, such as acetone and methyl ethyl ketone; esters, such as methyl acetate, ethyl acetate, isopropyl acetate, and butyl acetate; amides, such as N,N-dimethylformamide, N,N-dimethylacetamide (DMA), and N-methylpyrrolidone; nitriles, such as acetonitrile and propionitrile; acyclic or cyclic ethers, such as diethyl ether, dibutyl ether, dimethoxyethane, dioxane, and tetrahydrofuran; and organic acids, such as acetic acid. One type of these may be used alone, or two or more types thereof may be used in combination.

In particular, the solvent is preferably a mixture liquid of water and an amide (especially, DMA). The volume ratio of the water with respect to the total volume of the water and the amide is, for example, from 5 to 95 vol%. From the viewpoint of yielding a three-dimensional network structured metal complex crystal with large cavities, the lower limit value of the volume ratio is preferably 10 vol%, and particularly preferably 15 vol%, and the upper limit value of the volume ratio is preferably 80 vol%, particularly preferably 60 vol%, more preferably 50 vol%, even more preferably 40 vol%, particularly preferably 30 vol%, most preferably 25 vol%, and especially preferably 17 vol%.

The reaction temperature is, for example, from 60 to 100°C, preferably from 70 to 90°C, and particularly preferably from 80 to 90°C.

The reaction time is, for example, from 1 to 10 days, preferably from 3 to 10 days, and particularly preferably from 5 to 8 days.

The reaction atmosphere is not particularly limited as long as the reaction is not inhibited, and may be, for example, any of an air atmosphere, a nitrogen atmosphere, and an argon atmosphere.

After completion of the reaction, the obtained reaction product may be subjected to separation and purification by ordinary precipitation, washing, and filtration.

### Method for Determining Molecular Structure of Organic Compound (X)

The method for determining a molecular structure of the organic compound (X) includes the following step 1 and step 2:
step 1: allowing the organic compound (X) to infiltrate into the metal complex crystal, and fixing the organic compound (X) in cavities of the metal complex crystal; and
step 2: irradiating the metal complex crystal containing the fixed organic compound (X) with X-rays, analyzing obtained diffraction data, and determining a molecular structure of the organic compound (X).

The organic compound (X) is, for example, a compound having at least one functional group capable of interacting with an amide group (CONH group) contained in the metal complex crystal or a CO group or an NH group contained in the amide group through hydrogen bonding.

Examples of the functional group capable of interacting with at least one group selected from the CONH group, the CO group, or the NH group include groups having electronegativity of 1.0 to 4.0 (preferably 1.6 to 3.6, and particularly preferably 2.0 to 3.4).

Examples of the functional group include a substituted or unsubstituted amino group, a carboxyl group, an active methylene group, a nitrile group, a ketone group, an aldehyde group, an ester group, an ether group, an amide group, a hydroxy group, a halogen group, and a sulfonamide group.

The molecular weight of the organic compound (X) is, for example, 1000 or less, preferably 800 or less, and particularly preferably 600 or less. The lower limit value of the molecular weight of the organic compound (X) is, for example, 15, preferably 30, and particularly preferably 50.

The organic compound (X) is preferably a hydrophilic compound, and the log P value (octanol/water partition coefficient) of the organic compound (X) is, for example, -5 or greater and 7 or less. From the viewpoint of improving a rate of successful determination of the molecular structure, the lower limit value of the log P is preferably -3, and particularly preferably -1, and the upper limit value of the log P is preferably 6, particularly preferably 5, most preferably 4.5, and especially preferably 3.

Note that the log P value of the organic compound (X) can be determined by using a commercially available software (e.g., "EPI suite", the software jointly developed by United States Environmental Protection Agency (US EPA) (The Estimations Programs Interface for Windows) and SRC, Inc.).

The organic compound (X) is preferably a compound having no phenolic hydroxy group.

### (Step 1)

The step 1 is a step of allowing the organic compound (X) to infiltrate into the metal complex crystal, and fixing the organic compound (X) in cavities of the metal complex crystal.

From the viewpoint of improving the rate of successful determination of a molecular structure, as the metal complex crystal, a metal complex crystal having excellent transparency and a clear external shape (examples of the shape include needle-like shape, block-like shape, and flake-like shape) is preferably selected and used among the metal complex crystals obtained by the method for producing the metal complex crystal described above.

When the metal complex crystal is a particle having a needle-like or block-like shape, the length of the metal complex crystal is, for example, 10 µm or greater, preferably from 10 to 200 µm, and particularly preferably from 10 to 100 µm. When the metal complex crystal is too small, the measurement time tends to be longer. When the metal complex crystal is too large, adequate infiltration of the organic compound (X) becomes difficult to be achieved, and the rate of successful determination of the molecular structure tends to decrease.

The used amount of the metal complex crystal is, for example, from 0.1 to 50 mg, preferably from 0.5 to 15 mg, and particularly preferably from 1 to 3 mg.

The method for allowing an organic compound (X) to infiltrate into the metal complex crystal and fixing the organic compound (X) in cavities of the metal complex crystal is preferably appropriately adjusted depending on the state (liquid or solid) of the organic compound (X).

When the organic compound (X) is a liquid, examples of the fixing method include a method including placing the metal complex crystal in a container such as a test tube, and then adding the organic compound (X) to the container and allowing the container to stand still at -20 to 100°C for 0.25 to 24 hours.

Furthermore, when the organic compound (X) is a liquid, the purity may be increased in advance by subjecting the organic compound (X) to, for example, a separation treatment by liquid chromatography, and then, the organic compound (X) may be added to a container, such as a test tube.

When the organic compound (X) is a solid, examples of the fixing method include a method including placing the metal complex crystal in a container such as a test tube, and then adding a solution containing the organic compound (X) dissolved in a solvent inactive to the organic compound (X) (hereinafter, may be referred to as "inert solvent") to the container, and allowing the container to stand still at -20 to 100°C for 0.25 to 24 hours. Furthermore, after the still standing, the metal complex crystal into which the organic compound (X) is infiltrated may be subjected to a treatment, such as drying, and thus, the solution infiltrated into the metal complex crystal may be concentrated. By performing the concentration treatment, the rate of fixing the organic compound (X) in the cavities is increased, and the rate of successful determination of a molecular structure is improved.

The metal complex crystal has excellent solvent resistance, and the crystal structure can be maintained in various solvents. Thus, among inert solvents, a solvent that improves the rate of successful determination of a molecular structure can be selected and used. Examples of the inert solvent include alcohols such as methanol, ethanol, and isopropyl alcohol; and acyclic or cyclic ethers such as tetrahydrofuran (THF), dimethoxyethane, and dioxane. One type of these may be used alone, or two or more types thereof may be used in combination.

When the organic compound (X) is a solid, the concentration of the organic compound (X) in the solution obtained by dissolving the organic compound (X) in the inert solvent is, for example, from 0.001 to 50 µg/µL, preferably from 0.01 to 5 µg/µL, and more preferably from 0.1 to 1 µg/µL.

Because the metal complex crystal includes an amide group, when the organic compound (X) infiltrates into the metal complex crystal, the organic compound (X) incorporated into the cavities of the metal complex crystal can be firmly fixed by hydrogen bonding.

Through this step, an X-ray crystallographic sample, in which the organic compound (X) is fixed in the cavities of the metal complex crystal, is obtained.

### (Step 2)

The step 2 is a step of irradiating the X-ray crystallographic sample obtained in the step described above (i.e., the metal complex crystal containing the organic compound (X) fixed in the cavities of the metal complex crystal) with X-rays, analyzing obtained diffraction data, and determining a molecular structure of the organic compound (X).

In the method for determining a molecular structure of an organic compound (X), the organic compound (X) is not required to be crystallized, and thus, even when the amount of the organic compound (X) is too small to yield a single crystal or even when the organic compound (X) is a compound that cannot form a single crystal, the molecular structure can be determined by X-ray crystallography.

The method for determining a molecular structure of an organic compound (X) can be suitably used for determination of a molecular structure of a very small amount of impurity in pharmaceuticals, a food additive, and a very small amount of a component in animals and plants, for example.

The configurations and their combinations of the present disclosure described above are merely examples, and addition, omission, substitution, and modification of the configurations may be appropriately made without departing from the gist of the present disclosure. Each embodiment disclosed in the present specification can be combined with any other feature disclosed herein. Furthermore, the present disclosure is not limited to the embodiments.

### Examples

The present disclosure will next be described in more detail by way of Examples, but the present disclosure is not limited to Examples.

The single crystal X-ray crystallography was performed by using the following automated single crystal X-ray diffraction system.
Automated single crystal X-ray diffraction system: "XtaLAB SynergyCustom", available from Rigaku Holdings Corporation
Ray source: Cu-Kα line (wavelength: 0.79 Å)
Output: 50 mA, 24 kV

### Example 1 (Production of Metal Complex Crystal 1)

In a 10 mL test tube, a carboxylic acid-based ligand represented by Formula (c1-1-1) below (18 mg, 0.1 mol), a pyridine-based ligand represented by Formula (p1-2) below (59 mg, 0.2 mol), Zn(NO₃)₂6H₂O (62 mg, 0.1 mol), and a DMA/H₂O (6 mL/2 mL) mixed solvent were charged and dissolved by ultrasonic treatment.

Then, the test tube was capped tightly and allowed to stand still at 85°C for 6 days.

A crystal deposited on the test tube wall was subjected to ultrasonic irradiation and crushed to a size of approximately 100 to 200 µm.

A procedure in which the solvent in the test tube was removed by a Pasteur pipette and 3 mL of DMA was added, was repeated for 3 times. Thus, needle-like or block-like single crystal particles of a metal complex crystal 1 having DMA in the cavities were obtained.

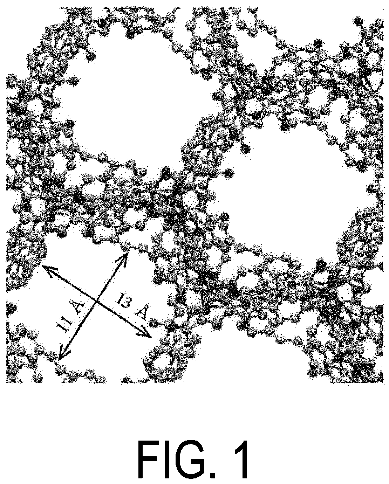

The crystal structure was confirmed by single crystal X-ray crystallography of the obtained metal complex crystal 1. A schematic view of the metal complex crystal 1 is illustrated in FIG. 1.

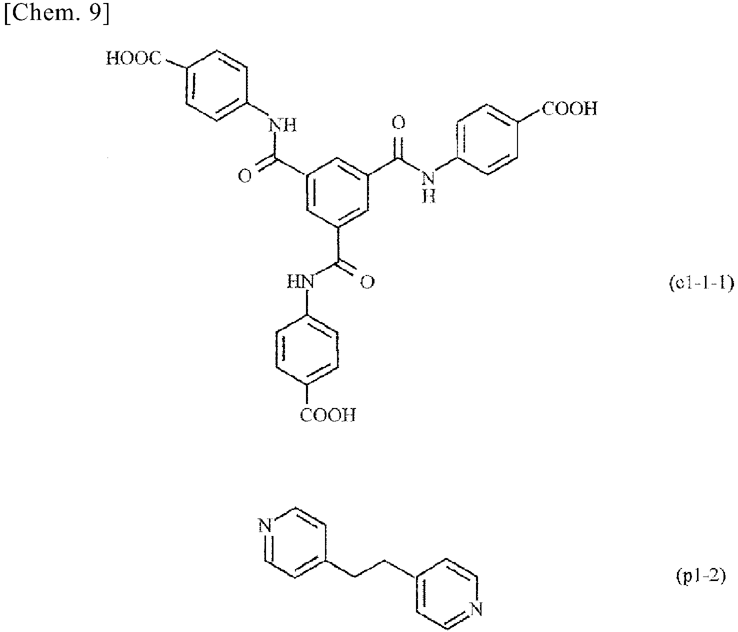

The single crystal X-ray crystallography showed that, in the crystal structure of the metal complex crystal 1, an infinite network having, as a repeating unit, a structure including one molecule of the carboxylic acid-based ligand represented by Formula (c1-1-1) above, two molecules of the pyridine-based ligand represented by Formula (p1-2) above, and three zinc ions was formed.

### Example 2 (Production of Metal Complex Crystal 2)

Single crystal particles of a metal complex crystal 2 were obtained by the same procedure as in Example 1 except for using a carboxylic acid-based ligand represented by Formula (c2-1-1) below in place of the carboxylic acid-based ligand represented by Formula (c1-1-1) above and using the pyridine-based ligand represented by Formula (p3-1) below in place of the pyridine-based ligand represented by Formula (p1-2) above.

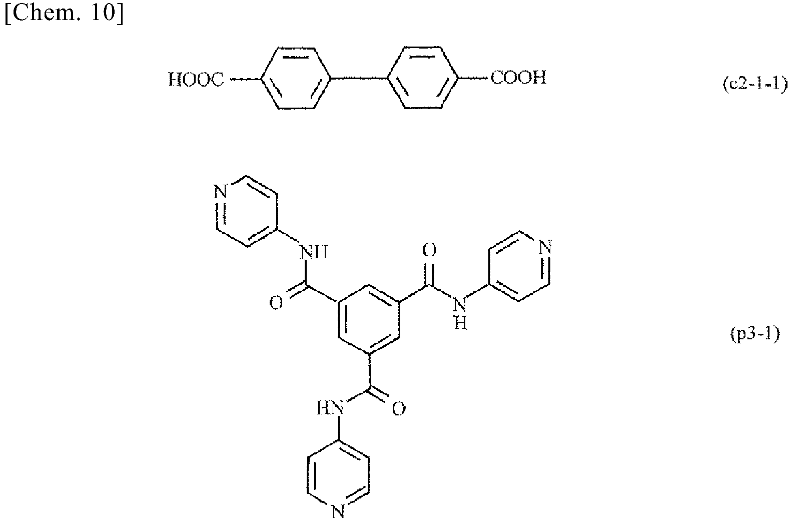

### Comparative Example 1 (Production of Metal Complex Crystal 3)

In a 50 mL vial, 2,4,6-tris(4-pyridyl)-1,3,5-triazine (TPT) (31.3 mg, 0.1 mmol), 21 mL of CHCl₃, and 1.7 mL of methanol were added and completely dissolved by ultrasonic treatment. In a screw-cap test tube, 4.5 mL of the solution thus obtained was charged.

Next, in a 10 mL vial, zinc chloride (20.5 mg, 0.15 mmol) and 5 mL of methanol were added and dissolved by ultrasonic treatment. In the screw-cap test tube, 1 mL of the solution thus obtained was added.

Then, the screw-cap test tube was capped and allowed to stand still at room temperature for 3 days. Thus, single crystal particles of a metal complex crystal 3 were obtained.

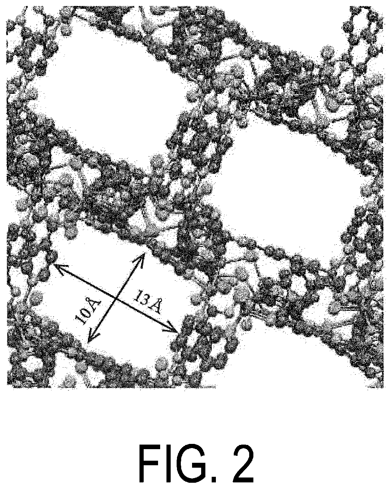

The single crystal X-ray crystallography of the obtained metal complex crystal 3 was performed. A schematic view of the metal complex crystal 3 is illustrated in FIG. 2.

### Solvent Resistance Evaluation

Solvent resistance was evaluated for the metal complex crystal 1 obtained in Example 1 and the metal complex crystal 3 obtained in Comparative Example 1 by the following method.

In a microvial, one particle of single crystal particle of each of the metal complex crystals was charged, 1 µL of the following solvent was added to the microvial and the microvial was allowed to stand still at 50°C or room temperature (25°C) for 17 hours, and whether the crystal structure was maintained or not after the still standing was visually observed. Then, the solvent resistance was evaluated based on the following criteria.

### Solvent Resistance Evaluation Criteria

Excellent (∘): The crystal structure could be maintained, and the framework of the metal complex crystal could be observed by single crystal X-ray measurement.
Good (Δ): The crystal structure could not be not maintained in the case of the standing at 50°C for 17 hours; and however, the crystal structure could be maintained in the case of the standing at room temperature for 17 hours.
Poor (×): The crystal structure could not be maintained.

### [Table 1]

**Table 1**

| | Metal complex crystal 1 | Metal complex crystal 3 |
|---|---|---|
| Methanol | ○ | × |
| Acetonitrile | ○ | × |
| Acetone | ○ | ○ |
| Ethyl acetate | ○ | ○ |
| Chloroform | ○ | ○ |

### Example 11 (Determination of Structure of Organic Compound Having Ketone Group)

In a microvial with a septum cap, 45 µL of THF was added, and then one particle of the single crystal particle of the metal complex crystal 1 obtained in Example 1 was immersed in THF. Subsequently, THF was removed as much as possible using a pipette.

Subsequently, 1 µg of 4-methylacetophenone was added in the microvial. Thereafter, the microvial was capped and allowed to stand still in a thermostatic chamber at 50°C for 17 hours.

Thereafter, the metal complex crystal 1 was removed from the microvial. The metal complex crystal 1 removed from the microvial was used as an X-ray crystallographic sample.

The obtained X-ray crystallographic sample was mounted on an X-ray diffractometer and analyzed for the crystal structure, and the ability to be analyzed for the crystal structure was evaluated based on the following evaluation criteria. The result is presented in the following table.

### Evaluation Criteria for Ability to be analyzed for Crystal Structure

⊚ (Excellent): The molecular structure could be determined very clearly.
○ (Good): The molecular structure could be determined with a certain degree of accuracy.
× (Failed): The molecular structure could not be determined.

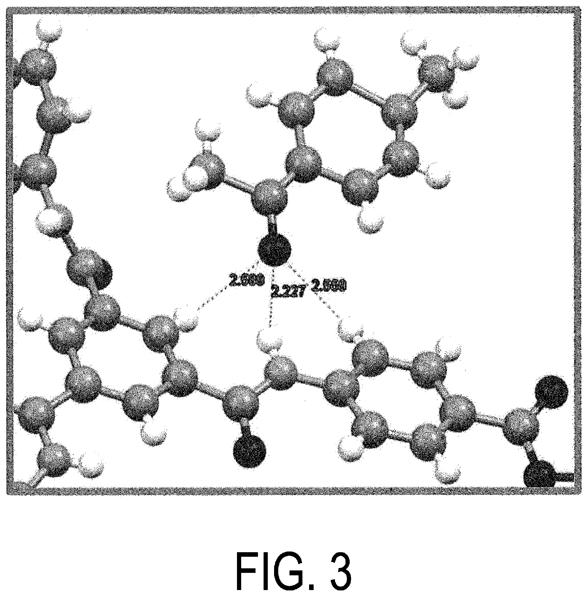

Furthermore, from the crystallographic result, it was confirmed that total of three hydrogen atoms, including one hydrogen atom of the amide group of the metal complex crystal 1 and two hydrogen atoms bonded to the benzene rings, formed hydrogen bonding with the carbonyl oxygen of 4-methylacetophenone (i.e., the oxygen atom contained in carbonyl). The schematic view of the crystal structure of the X-ray crystallographic sample is illustrated in FIG. 3.

### Examples 12 and 13 (Determination of Structure of Organic Compound Having Ketone Group)

The same procedure as in Example 11 was performed except for using 1,3-diphenyl-2-propanone or 5-nonanone in place of 4-methylacetophenone. The results are presented in the following table.

### Comparative Examples 11 to 13 (Determination of Structure of Organic Compound Having Ketone Group)

The same procedures as in Examples 11 to 13 were performed except for using the metal complex crystal 3 in place of the metal complex crystal 1. The results are presented in the following table.

### [Table 2]

**Table 2**

| No. | Organic compound (X) | Log P value | Metal complex crystal 1 | Metal complex crystal 3 |
|---|---|---|---|---|
| 11 | 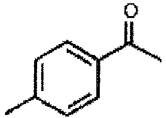 | 2.10 | ○ | ○ |
| 12 | 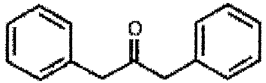 | 3.18 | ⊚ | × |
| 13 | 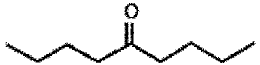 | 2.88 | ○ | × |

### Example 14 (Determination of Structure of Organic Compound Having Amide Group)

The same procedure as in Example 11 was performed except for using 1,3-dimethyl-2-imidazolidinone in place of 4-methylacetophenone, and thus an X-ray crystallographic sample was obtained.

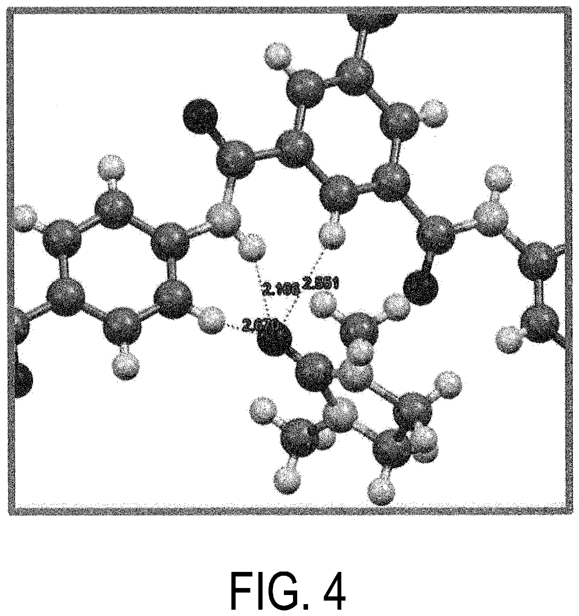

When the obtained X-ray crystallographic sample was mounted on an X-ray diffractometer and crystallography was performed, it was confirmed that total of three hydrogen atoms, including one hydrogen atom of the amide group of the metal complex crystal 1 and two hydrogen atoms bonded to the benzene rings, formed hydrogen bonding with the carbonyl oxygen of 1,3-dimethyl-2-imidazolidinone. The schematic view of the crystal structure of the X-ray crystallographic sample is illustrated in FIG. 4.

### Example 15 (Determination of Structure of Organic Compound Having Amide Group)

The same procedure as in Example 14 was performed except for using N,N-dimethylacetamide in place of 1,3-dimethyl-2-imidazolidinone. The result is presented in the following table.

### Comparative Examples 14 and 15 (Determination of Structure of Organic Compound Having Amide Group)

The same procedures as in Examples 14 and 15 were performed except for using the metal complex crystal 3 in place of the metal complex crystal 1. The results are presented in the following table.

### [Table 3]

**Table 3**

| No. | Organic compound (X) | Log P value | Metal complex crystal 1 | Metal complex crystal 3 |
|---|---|---|---|---|
| 14 | 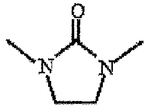 | -0.31 Estimated value | ○ | × *¹ |
| 15 | 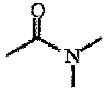 | -0.77 | ○ | × *¹ |

| | | | | |
|---|---|---|---|---|
| *1 The crystal structure of the metal complex crystal 3 was collapsed. | | | | |

### Example 16 (Determination of Structure of Organic Compound Having Ester Group)

The same procedure as in Example 11 was performed except for using methyl 2-furancarboxylate (log P value: 1.00 (database match)) in place of 4-methylacetophenone, and thus an X-ray crystallographic sample was obtained.

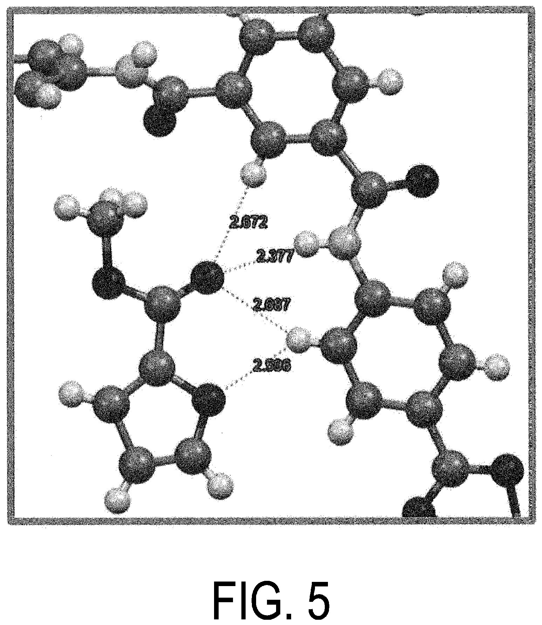

When the obtained X-ray crystallographic sample was mounted on an X-ray diffractometer and crystallography was performed, it was confirmed that total of three hydrogen atoms, including one hydrogen atom of the amide group of the metal complex crystal 1 and two hydrogen atoms bonded to the benzene rings, formed hydrogen bonding with the carbonyl oxygen of methyl 2-furancarboxylate, and the oxygen atom of the furan ring also formed hydrogen bonding with the hydrogen atom bonded to the benzene ring. The schematic view of the crystal structure of the X-ray crystallographic sample is illustrated in FIG. 5.

### Examples 17 and 18 (Determination of Structure of Organic Compound Having Ester Group)

The same procedure as in Example 16 was performed except for using γ-butyrolactone (log P value: -0.64 (database match)) or phthalide (log P value: 0.80 (database match)) in place of methyl 2-furancarboxylate.

As a result, the entire molecular structures of all the organic compounds could be determined by X-ray diffraction images.

Furthermore, for methyl 2-furancarboxylate, it was confirmed that the carbonyl oxygen interacted with total of three hydrogen atoms, including one hydrogen atom of the amide group of the metal complex crystal 1 and two hydrogen atoms bonded to the benzene rings, and was fixed in the cavity of the metal complex crystal 1.

It was confirmed that, for γ-butyrolactone, the carbonyl oxygen interacted with total of three hydrogen atoms, including one hydrogen atom of the amide group of the metal complex crystal 1 and two hydrogen atoms bonded to the benzene rings, and was fixed in the cavity of the metal complex crystal 1.

### Example 19 (Determination of Structure of Organic Compound Having Hydroxy Group, Ether Group, or Aldehyde Group)

The same procedure as in Example 11 was performed except for using 2,4-dimethoxybenzyl alcohol (log P value: 1.24) in place of 4-methylacetophenone, and thus an X-ray crystallographic sample was obtained.

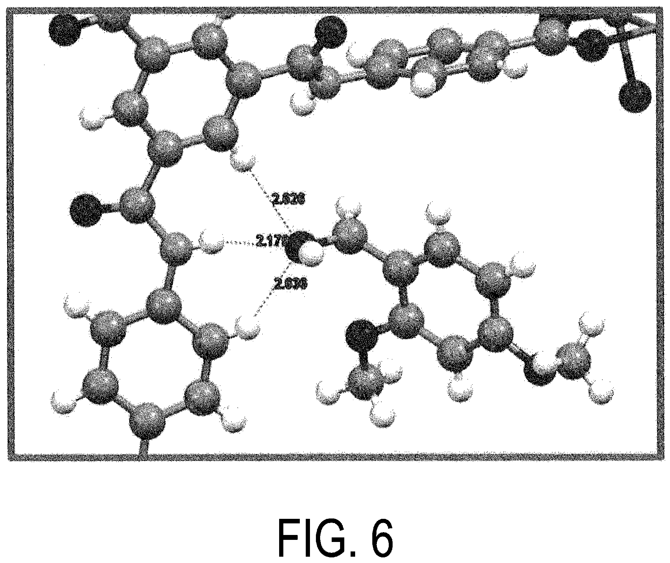

When the obtained X-ray crystallographic sample was mounted on an X-ray diffractometer and crystallography was performed, it was confirmed that total of three hydrogen atoms, including one hydrogen atom of the amide group of the metal complex crystal 1 and two hydrogen atoms bonded to the benzene rings, formed hydrogen bonding with the hydrogen atom contained in the hydroxy group of 2,4-dimethoxybenzyl alcohol. The schematic view of the crystal structure of the X-ray crystallographic sample is illustrated in FIG. 6.

### Examples 20 and 21 (Determination of Structure of Organic Compound Having Hydroxy Group, Ether Group, or Aldehyde Group)

The same procedure as in Example 19 was performed except for using 2,4-dimethyl-1,3-dioxane (log P value: 1.02 (estimated value)) or 1-naphthaldehyde (log P value: 2.89 (estimated value)) in place of 2,4-dimethoxybenzyl alcohol, for the organic compound.

As a result, the entire molecular structures of all the organic compounds could be determined by X-ray diffraction images.

Furthermore, it was confirmed that, for 2,4-dimethyl-1,3-dioxane, the oxygen atom at the 3-position interacted with total of three hydrogen atoms, including one hydrogen atom of the amide group of the metal complex crystal 1 and two hydrogen atoms bonded to the benzene rings, and was fixed in the cavity of the metal complex crystal 1.

It was confirmed that, for 1-naphthaldehyde, the oxygen atom contained in the aldehyde group interacted with total of three hydrogen atoms, including one hydrogen atom of the amide group of the metal complex crystal 1 and two hydrogen atoms bonded to the benzene rings, and was fixed in the cavity of the metal complex crystal 1.

### Example 22 (Determination of Structure of Organic Compound Having Carboxyl Group)

In a microvial with a septum cap, 45 µL of THF was charged, and then one particle of the single crystal of the metal complex crystal 1 containing DMA obtained in Example 1 was immersed in THF.

2-naphthalenecarboxylic acid (log P value: 3.28 (database match)) was dissolved in THF such that the concentration of 2-naphthalenecarboxylic acid was 1 µg/ 1 µL, and 5 µL of the obtained sample solution (containing 5 µg of 2-naphthalenecarboxylic acid) was added in the microvial.

Subsequently, the microvial was capped, and a pinhole was made on the septum part by using an injection needle having a needle diameter of 0.8 mm. The microvial was allowed to stand still in a thermostatic chamber at 50°C for 17 hours.

Thereafter, the metal complex crystal 1 was removed from the microvial. The metal complex crystal 1 removed from the microvial was used as an X-ray crystallographic sample.

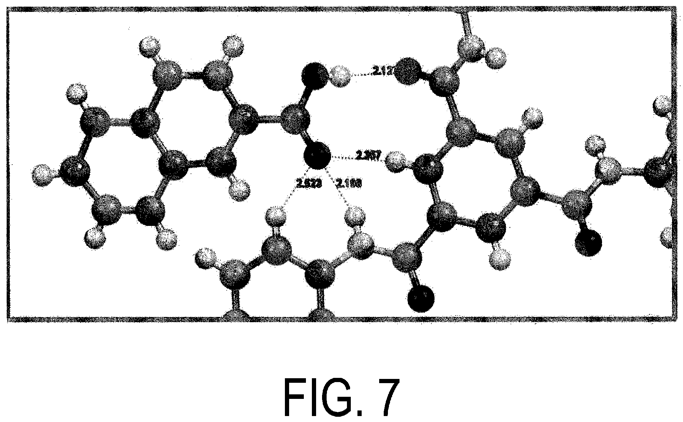

When the obtained X-ray crystallographic sample was mounted on an X-ray diffractometer and crystallography was performed, it was confirmed that total of three hydrogen atoms, including one hydrogen atom of the amide group of the metal complex crystal 1 and two hydrogen atoms bonded to the benzene rings, formed hydrogen bonding with the carbonyl oxygen of 2-naphthalenecarboxylic acid. The schematic view of the crystal structure of the X-ray crystallographic sample is illustrated in FIG. 7.

### Example 23 (Determination of Structure of Organic Compound Having Sulfonamide Group)

The same procedure as in Example 22 was performed except for using zonisamide (log P value: 0.36 (database match)) in place of 2-naphthalenecarboxylic acid, and thus an X-ray crystallographic sample was obtained.

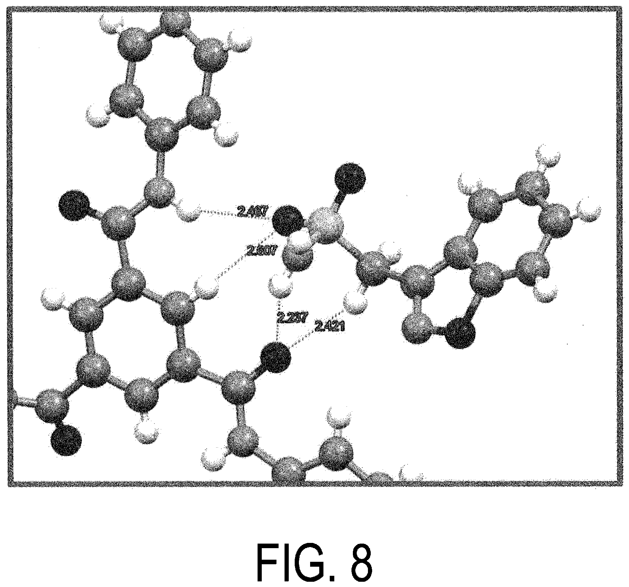

When the obtained X-ray crystallographic sample was mounted on an X-ray diffractometer and crystallography was performed, it was confirmed that total of three hydrogen atoms, including one hydrogen atom of the amide group of the metal complex crystal 1 and two hydrogen atoms bonded to the benzene rings, formed hydrogen bonding with the sulfone moiety, the amine moiety and the hydrogen atom at the α-position of the sulfonamide group of zonisamide. The schematic view of the crystal structure of the X-ray crystallographic sample is illustrated in FIG. 8.

### Example 24 (Determination of Structure of Organic Compound Having Amino Group)

The same procedure as in Example 11 was performed except for using 2,4,6-trimethylaniline (log P value: 2.72) in place of 4-methylacetophenone, and thus an X-ray crystallographic sample was obtained.

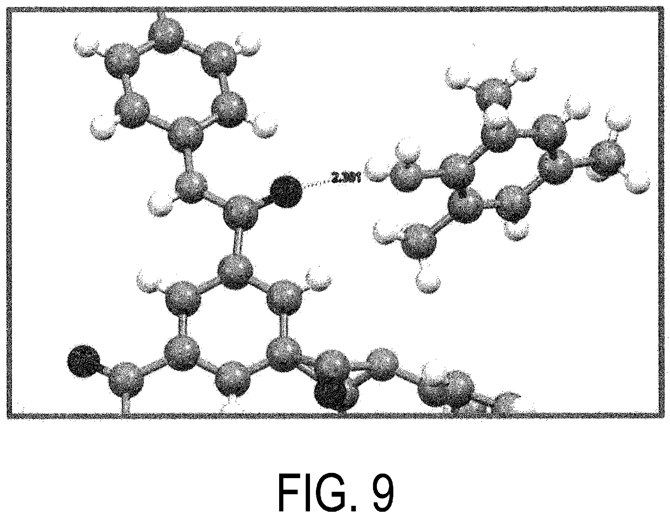

When the obtained X-ray crystallographic sample was mounted on an X-ray diffractometer and crystallography was performed, it was confirmed that the carbonyl oxygen of the metal complex crystal 1 formed hydrogen bonding with the N-H group of 2,4,6-trimethylaniline. The schematic view of the crystal structure of the X-ray crystallographic sample is illustrated in FIG. 9.

### Example 25 (Determination of Structure of Bergapten)

The same procedure as in Example 22 was performed except for using bergapten (log P value: 1.93 (database match)) in place of 2-naphthalenecarboxylic acid, and thus an X-ray crystallographic sample was obtained.

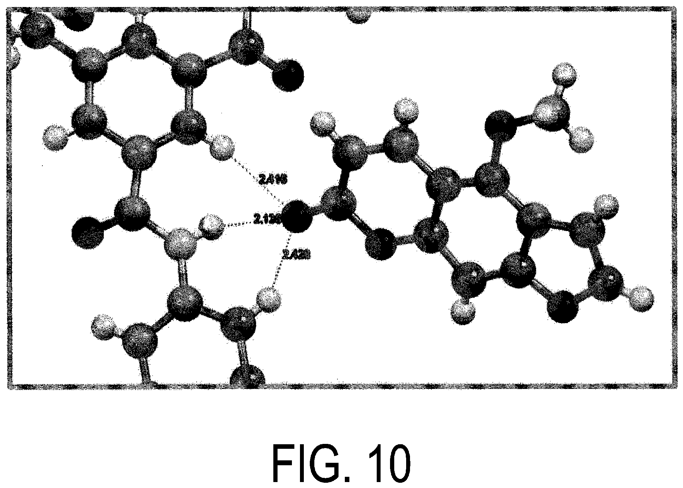

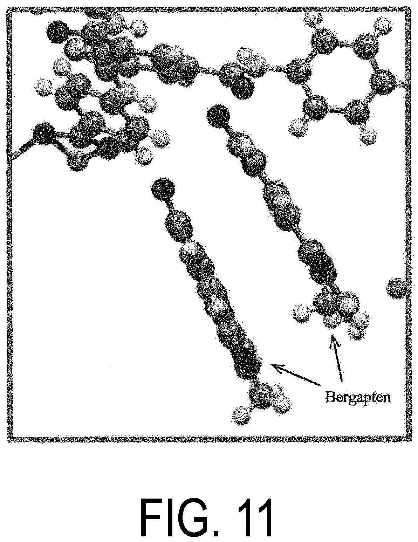

When the obtained X-ray crystallographic sample was mounted on an X-ray diffractometer and crystallography was performed, it was confirmed that total of three hydrogen atoms, including one hydrogen atom of the amide group of the metal complex crystal 1 and two hydrogen atoms bonded to the benzene rings, formed hydrogen bonding with the oxygen atom of the ester moiety of bergapten, and two molecules of the bergapten exhibited a Π-Π interaction. The schematic views of the crystal structure of the X-ray crystallographic sample are illustrated in FIG. 10 and FIG. 11.

### Example 26 (Determination of Structure of 1-Aminopyrene)

The same procedure as in Example 22 was performed except for using 1-aminopyrene (log P value: 4.31 (database match)) in place of 2-naphthalenecarboxylic acid, and thus an X-ray crystallographic sample was obtained.

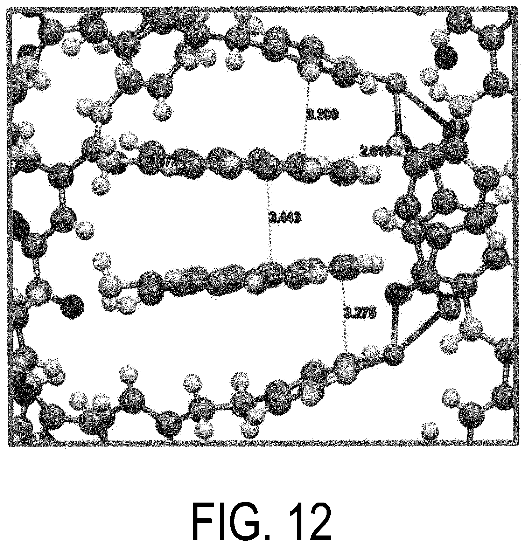

When the obtained X-ray crystallographic sample was mounted on an X-ray diffractometer and crystallography was performed, it was confirmed that the metal complex crystal 1 and 1-aminopyrene exhibit Π-Π interactions. The schematic view of the crystal structure of the X-ray crystallographic sample is illustrated in FIG. 12.

### Example 27

The same procedure as in Example 11 was performed except for using 1-fluoronaphthalene (log P value: 3.37 (database match)) in place of 4-methylacetophenone. As a result, for all of the organic compounds, the organic compounds were fixed in the cavities of the metal complex crystal 1 utilizing the Π-Π interaction with the metal complex crystal 1, and the entire molecular structures were determined by X-ray diffraction images.

### Example 28

The same procedure as in Example 22 was performed except for using 2-triphenylenecarboxaldehyde in place of 2-naphthalenecarboxylic acid. As a result, for all of the organic compounds, the organic compounds were fixed in the cavities of the metal complex crystal 1 utilizing the Π-Π interaction with the metal complex crystal 1, and the entire molecular structures were determined by X-ray diffraction images.

To summarize the above, configurations and variations of the present disclosure are additionally described below.
[1] A metal complex crystal containing a metal ion and a ligand coordinated to the metal ion,
   the metal complex crystal having a three-dimensional network structure having regularly arranged cavities,
   the ligand containing a carboxylic acid-based ligand (c) and a pyridine-based ligand (p), and
   the carboxylic acid-based ligand (c) and the pyridine-based ligand (p) being in a combination selected from [I] to [IV]:
      [I] a compound represented by Formula (c1) and a compound represented by Formula (p1);
      [II] a compound represented by Formula (c1) and a compound represented by Formula (p2);
      [III] a compound represented by Formula (c2) and a compound represented by Formula (p2); and
      [IV] a compound represented by Formula (c2) and a compound represented by Formula (p3).
[2] The metal complex crystal according to [1], wherein the metal ion is a metal ion selected from a zinc ion, an iron ion, a cobalt ion, a nickel ion, a copper ion, or a silver ion.
[3] The metal complex crystal according to [1] or [2], wherein the compound represented by Formula (c1) is at least one compound selected from a compound represented by Formula (c1-1), a compound represented by Formula (c1-2), a compound represented by Formula (c1-3), or a compound represented by Formula (c1-4).
[4] The metal complex crystal according to any one of [1] to [3], wherein the compound represented by Formula (c1) is the compound represented by Formula (c1-1) and/or the compound represented by Formula (c1-2).
[5] The metal complex crystal according to any one of [1] to [4], wherein the compound represented by Formula (c1) is the compound represented by Formula (c1-1) and/or the compound represented by Formula (c1-3).
[6] The metal complex crystal according to any one of [1] to [5], wherein the compound represented by Formula (c2) is at least one compound selected from a compound represented by Formula (c2-1), a compound represented by Formula (c2-2), a compound represented by Formula (c2-3), a compound represented by Formula (c2-4), or a compound represented by Formula (c2-5).
[7] The metal complex crystal according to any one of [1] to [5], wherein the compound represented by Formula (c2) is the compound represented by Formula (c2-1) and/or the compound represented by Formula (c2-2).
[8] The metal complex crystal according to any one of [1] to [7], wherein the compound represented by Formula (p1) is at least one compound selected from a compound represented by Formula (p1-1), a compound represented by Formula (p1-2), a compound represented by Formula (p1-3), a compound represented by Formula (p1-4), a compound represented by Formula (p1-5), a compound represented by Formula (p1-6), or a compound represented by Formula (p1-7).
[9] The metal complex crystal according to any one of [1] to [7], wherein the compound represented by Formula (p1) is at least one compound selected from the compound represented by Formula (p1-1), the compound represented by Formula (p1-2), the compound represented by Formula (p1-5), or the compound represented by Formula (p1-6).
[10] The metal complex crystal according to any one of [1] to [7], wherein the compound represented by Formula (p1) is the compound represented by Formula (p1-2).
[11] The metal complex crystal according to any one of [1] to [10], wherein the compound represented by Formula (p3) is at least one compound selected from a compound represented by Formula (p3-1), a compound represented by Formula (p3-2), a compound represented by Formula (p3-3), or a compound represented by Formula (p3-4).
[12] The metal complex crystal according to any one of [1] to [10], wherein the compound represented by Formula (p3) is the compound represented by Formula (p3-1) and/or the compound represented by Formula (p3-2).
[13] The metal complex crystal according to any one of [1] to [10], wherein the compound represented by Formula (p3) is the compound represented by Formula (p3-1) and/or the compound represented by Formula (p3-3).
[14] The metal complex crystal according to any one of [1] to [13], wherein a content ratio of the carboxylic acid-based ligand (c) to the pyridine-based ligand (p) [carboxylic acid-based ligand (c)/pyridine-based ligand (p); molar ratio] is from 30/70 to 70/30.
[15] The metal complex crystal according to any one of [1] to [14], wherein the ligand contains the compound represented by Formula (c1) and the compound represented by Formula (p1), and the compound represented by Formula (c1)/the compound represented by Formula (p1) (molar ratio) is from 30/70 to 70/30.
[16] The metal complex crystal according to any one of [1] to [15], wherein the ligand contains the compound represented by Formula (c2) and the compound represented by Formula (p2), and the compound represented by Formula (c2)/the compound represented by Formula (p2) (molar ratio) is from 30/70 to 70/30.
[17] The metal complex crystal according to any one of [1] to [16], wherein the ligand contains the compound represented by Formula (c2) and the compound represented by Formula (p3), and the compound represented by Formula (c2)/the compound represented by Formula (p3) (molar ratio) is from 30/70 to 70/30.
[18] The metal complex crystal according to any one of [1] to [17], wherein a proportion of a total content of the carboxylic acid-based ligand (c) and the pyridine-based ligand (p) with respect to a total amount of the ligand is 50 wt.% or greater.
[19] The metal complex crystal according to any one of [1] to [18], wherein a content ratio of the metal ion to the carboxylic acid-based ligand (c) [metal ion/carboxylic acid-based ligand (c); molar ratio] is from 30/70 to 70/30.
[20] The metal complex crystal according to any one of [1] to [19], wherein a content ratio of the metal ion to the pyridine-based ligand (p) [metal ion/pyridine-based ligand (p); molar ratio] is from 20/80 to 80/20.
[21] The metal complex crystal according to any one of [1] to [20], wherein the metal complex crystal is a metal complex crystal for molecular structure analysis of an organic compound.
[22] The metal complex crystal according to [21], wherein the organic compound is an organic compound including a functional group capable of interacting with a CO group, an NH group, or a CONH group.
[23] The metal complex crystal according to [21] or [22], wherein the organic compound is an organic compound having a log P value of -5 or greater and 7 or less.
[24] The metal complex crystal according to any one of [21] to [23], wherein the organic compound is an organic compound including at least one functional group selected from a substituted or unsubstituted amino group, a carboxyl group, an active methylene group, a nitrile group, a ketone group, an aldehyde group, an ester group, an ether group, an amide group, a hydroxy group, a halogen group, or a sulfonamide group.
[25] Use of the metal complex crystal described in any one of [1] to [20] as a material for forming an X-ray crystallographic sample for an organic compound.
[26] The use as a material for forming an X-ray crystallographic sample of an organic compound according to [25], wherein the organic compound is an organic compound including a functional group capable of interacting with a CO group, an NH group, or a CONH group.
[27] The use as a material for forming an X-ray crystallographic sample of an organic compound according to [25] or [26], wherein the organic compound is an organic compound having a log P value of -5 or greater and 7 or less.
[28] The use as a material for forming an X-ray crystallographic sample of an organic compound according to any one of [25] to [27], wherein the organic compound is an organic compound including at least one functional group selected from a substituted or unsubstituted amino group, a carboxyl group, an active methylene group, a nitrile group, a ketone group, an aldehyde group, an ester group, an ether group, an amide group, a hydroxy group, a halogen group, or a sulfonamide group.
[29] A method for determining a molecular structure of an organic compound (X), the method including steps 1 and 2:
   step 1: allowing the organic compound (X) to infiltrate into the metal complex crystal described in any one of [1] to [24], and fixing the organic compound (X) in cavities of the metal complex crystal; and
   step 2: irradiating the metal complex crystal containing the fixed organic compound (X) with X-rays, analyzing obtained diffraction data, and determining a molecular structure of the organic compound (X).

### Industrial Applicability

The metal complex crystal of the present disclosure is highly versatile as a material for forming an X-ray crystallographic sample of an organic compound (X) (especially, a hydrophilic organic compound).

## Claims

1. A metal complex crystal comprising a metal ion and a ligand coordinated to the metal ion,
the metal complex crystal having a three-dimensional network structure having regularly arranged cavities,
the ligand containing a carboxylic acid-based ligand (c) and a pyridine-based ligand (p), and
the carboxylic acid-based ligand (c) and the pyridine-based ligand (p) being in a combination selected from [I] to [IV]:
[I] a compound represented by Formula (c1) and a compound represented by Formula (p1);
[II] a compound represented by Formula (c1) and a compound represented by Formula (p2);
[III] a compound represented by Formula (c2) and a compound represented by Formula (p2); and
[IV] a compound represented by Formula (c2) and a compound represented by Formula (p3); 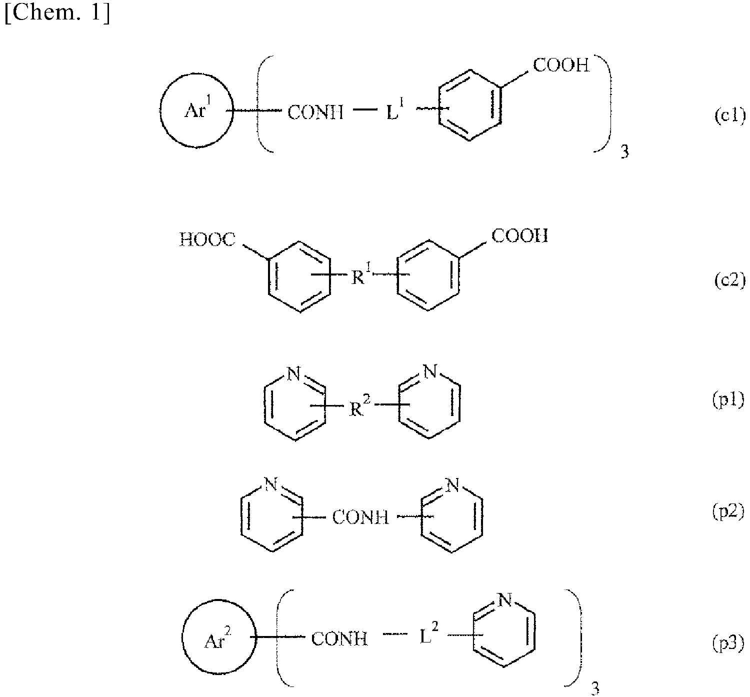
where, Ar¹ and Ar² are the same or different and each represent an aromatic ring; R¹ and R² are the same or different and each represent a group selected from a single bond, a divalent hydrocarbon group, a divalent heterocyclic group, or a divalent group containing two or more of the groups, the two or more of the groups being linked, and L¹ and L² are the same or different and each represent a single bond or a linking group.

2. The metal complex crystal according to claim 1, wherein the metal ion is a metal ion selected from a zinc ion, an iron ion, a cobalt ion, a nickel ion, a copper ion, or a silver ion.

3. The metal complex crystal according to claim 1 or 2, wherein the metal complex crystal is a metal complex crystal for molecular structure analysis of an organic compound.

4. The metal complex crystal according to claim 3, wherein the organic compound is an organic compound including a functional group capable of interacting with a CO group, an NH group, or a CONH group.

5. The metal complex crystal according to claim 3, wherein the organic compound is an organic compound having a log P value of -5 or greater and 7 or less.

6. The metal complex crystal according to claim 3, wherein the organic compound is an organic compound including at least one functional group selected from a substituted or unsubstituted amino group, a carboxyl group, an active methylene group, a nitrile group, a ketone group, an aldehyde group, an ester group, an ether group, an amide group, a hydroxy group, a halogen group, or a sulfonamide group.

7. A method for determining a molecular structure of an organic compound (X), the method comprising steps 1 and 2:
step 1: allowing the organic compound (X) to infiltrate into the metal complex crystal described in claim 1 or 2, and fixing the organic compound (X) in cavities of the metal complex crystal; and
step 2: irradiating the metal complex crystal containing the fixed organic compound (X) with X-rays, analyzing obtained diffraction data, and determining a molecular structure of the organic compound (X).
